# EUROPEAN PATENT APPLICATION

(11) **EP 3 473 179 A1**
(43) Date of publication of application: **24.04.2019**
(21) Application number: 17813220.5
(22) Date of filing: 08.06.2017
(51) Int. Cl.: A61B 5/154, A61J 1/05, G01N 1/10, G01N 33/48

(54) **EMULSION PRODUCTION MEMBER, AND SAMPLE COLLECTION TUBE AND BLOOD COLLECTION TUBE EACH EQUIPPED WITH SAME**

(30) Priority: 16.06.2016 JP 2016119764
(71) Applicant: Enplas Corporation, Kawaguchi-shi, Saitama 332-0034 (JP)
(72) Inventor: SUNAGA, Nobuya, Saitama 332-0034 (JP)
(74) Representative: Epping - Hermann - Fischer
(86) International application number: PCT/JP2017/021388
(87) International publication number: WO 2017/217323

(57) **Abstract**

The present application provides: an emulsion production member for dividing a sample into fine droplets and dispersing the fine droplets in a dispersion medium to produce an emulsion; and a sample collection tube and a blood collection tube, each of which is equipped with the emulsion production member. The emulsion production member is provided with: a plug body, a sample injection hole, a sample storage unit, a dispersion medium injection hole, and a dispersion medium storage unit. The emulsion production member is further provided with: a discharge port; a sample flow passage; and a dispersion medium flow passage for connecting the dispersion medium storage unit and the sample flow passage to each other to cause the dispersion medium to flow in order to divide the sample circulating in the sample flow path into fine droplets by means of the dispersion medium.

## Description

### Technical Field

The present invention relates to an emulsion production member, a sample collection tube including the emulsion production member, and a blood collection tube including the emulsion production member.

### Background Art

Collection of blood, bodily fluid or the like from human bodies or the like is performed in immunity inspection and various researches. A commonly used sample collection tube is sealed with a stopper composed of rubber or the like press-fitted in an opening of a container main body, and a blood collection tube or the like whose internal pressure is reduced is practically used to quickly collect blood or the like.

In addition, for example, a blood collection tube which includes a blood separation filter in the blood collection tube and is capable of separating blood into blood cell and plasma or serum is proposed (PTL 1).

### Citation List

### Patent Literature

PTL 1
Japanese Patent Application Laid-Open No. 2007-536

### Summary of Invention

### Technical Problem

However, with conventional blood collection tubes, it is difficult to separate a collected sample in a unit of a blood cell or a unit of a cell. For example, with the blood collection tube disclosed in PTL 1 and the like, it is difficult to separate blood cell or cell while blood can be separated into blood cell and plasma or serum. Therefore, when hemolysis occurs, DNA, RNA, and protein are mixed together regardless of blood cell origin or cell origin, and it is difficult to analyze a protein or a nucleic acid composed only of a specific cell strain.

If blood can be separately stored in a unit of cell or a unit of cell, a component originating from a specific cell can be identified even when hemolysis occurs. In view of this, the present invention provides an emulsion production member for establishing an emulsion state by dividing a sample into minute droplets, and dispersing the minute droplets with a disperse medium, and a blood collection tube and a sample collection tube using the emulsion production member.

### Solution to Problem

An emulsion production member of an embodiment of the present invention is configured to be attached to a container main body whose internal pressure is reduced, and configured to divide a sample into minute droplets and disperse the minute droplets with a disperse medium, the emulsion production member including: a stopper configured to maintain the reduced internal pressure of the container main body; a sample injection hole configured to inject the sample into the stopper; a sample housing part disposed in the stopper and configured to house the sample injected through the sample injection hole; a disperse medium injection hole configured to inject the disperse medium into the stopper; a disperse medium housing part disposed in the stopper and configured to house the disperse medium injected through the disperse medium injection hole; an outlet configured to discharge, to the container main body side, the sample housed in the sample housing part and the disperse medium housed in the disperse medium housing part; a sample channel configured to connect between the sample housing part and the outlet and carry the sample; and a disperse medium channel configured to connect between the disperse medium housing part and the sample channel, and configured to carry the disperse medium to divide the sample flowing in the sample channel into minute droplets by the disperse medium.

A sample collection tube of an embodiment of the present invention includes: a container main body including an opening; and the above-mentioned emulsion production member attached to the opening of the container main body. A pressure inside the container main body is in a reduced state.

A blood collection tube of an embodiment of the present invention includes: a container main body including an opening; and the above-mentioned emulsion production member attached to the opening of the container main body. The sample is blood.

### Advantageous Effects of Invention

With the emulsion production member of an embodiment of the present invention, a sample can be divided into minute droplets, and can be stored in a dispersed state with a disperse medium. Accordingly, even when hemolysis occurs in minute droplets in a case that the sample is blood for example, the components of the blood cell or the cell are not mixed with the components of other blood cells or other cells, and thus a component originating from a specific blood cell or a specific cell can be inspected, for example.

### Brief Description of Drawings

FIG. 1A is a front view of a sample collection tube using an emulsion production member of an embodiment of the present invention, FIG. 1B is a sectional view of the sample collection tube of FIG. 1A, and FIG. 1C is a plan view of the sample collection tube of FIG. 1A;
FIG. 2 is an exploded view of the emulsion production member of the embodiment of the present invention;
FIG. 3A is a front view of a first member of the emulsion production member of the embodiment of the present invention, FIG. 3B is a sectional view of the first member, and FIG. 3C is a plan view of the first member;
FIG. 4A is a front view of a second member of the emulsion production member of the embodiment of the present invention, FIG. 4B is a sectional view of the second member, and FIG. 4C is a bottom view of the second member;
FIG. 5 is a partially enlarged sectional view of a bottom surface of the second member of the emulsion production member of the embodiment of the present invention;
FIG. 6 is a partially enlarged sectional view of the bottom surface of the second member of the emulsion production member of the embodiment of the present invention; and
FIG. 7A is a front view of a film of the emulsion production member of the embodiment of the present invention, and FIG. 7B is a plan view of the film.

### Description of Embodiments

An embodiment of the present invention is described below in detail with reference to the accompanying drawings. The present invention is not limited to the embodiment described below.

### Configuration of emulsion production member

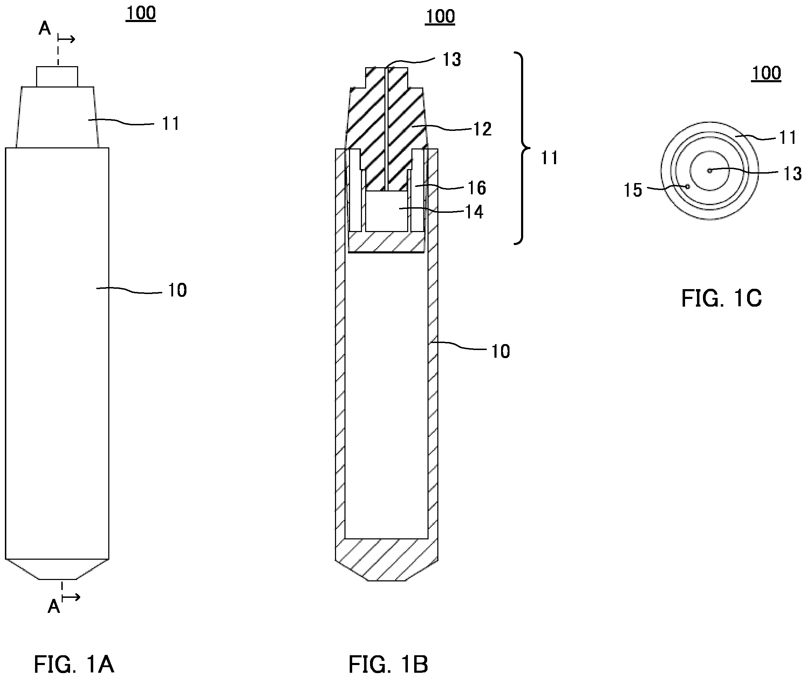

FIGS. 1A to 1C illustrate a configuration of sample collection tube 100 using emulsion production member 11 according to the present embodiment. FIG. 1A is a front view of sample collection tube 100, FIG. 1B is a sectional view of sample collection tube 100 taken along line A-A of FIG. 1A, and FIG. 1C is a plan view of sample collection tube 100 of FIG. 1A.

As illustrated in FIG. 1A, emulsion production member 11 of the present embodiment is attached to container main body 10 of sample collection tube 100. Emulsion production member 11 of the present embodiment functions as a stopper for hermetically sealing container main body 10 and for maintaining the reduced pressure state in container main body 10. As illustrated in FIG. 1B and FIG. 1C, emulsion production member 11 includes base part 12 for hermetically sealing container main body 10, sample injection hole 13 for injecting a sample into emulsion production member (base part) 11, and disperse medium injection hole 15 for injecting a disperse medium into emulsion production member (base part) 11. With this configuration, when a sample and a disperse medium are introduced into emulsion production member 11, emulsion in which the sample is divided by the disperse medium into minute droplets is produced.

Here, the sample which can be handled with emulsion production member 11 of the present embodiment is not limited as long as the sample is a liquid component, and examples of the sample include solutions of DNA, RNA, protein and the like, cell suspension, blood, bodily fluid, and the like. On the other hand, the disperse medium which can be handled with emulsion production member 11 of the present embodiment is appropriately selected in accordance with the type of the sample. The disperse medium is not limited as long as the disperse medium is not compatible with the sample and does not modify the sample. In a case that the sample is blood, for example, the disperse medium may be various oils such as mineral oil and silicone oil which are liquid at normal temperature. Alternatively, oil added with a surfactant may be used.

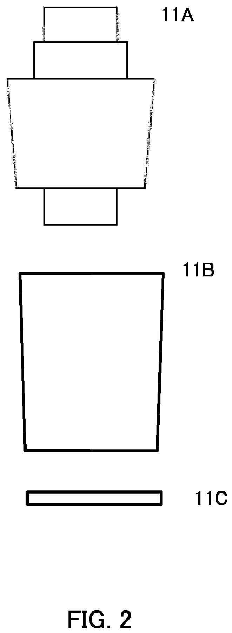

As illustrated in the exploded view of FIG. 2, emulsion production member 11 of the present embodiment includes three members, first member 11A, second member 11B, and film 11C. Note that, in emulsion production member 11 of the present invention, two or all of the three members may be integrated with each other. In addition, emulsion production member 11 of the present invention may be formed such that emulsion production member 11 can be divided into four or more members.

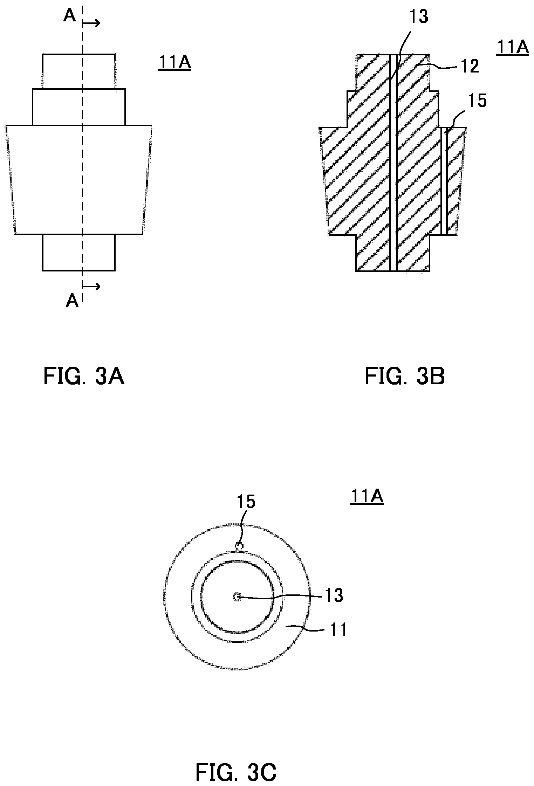

FIG. 3A is a front view of first member 11A, FIG. 3B is a sectional view of first member 11A taken along line A-A, and FIG. 3C is a plan view of first member 11A. As illustrated in FIG. 3B, first member 11A of the present embodiment includes base part 12. In the present embodiment, as illustrated in FIG. 1B, base part 12 is fitted to the inside of the opening of container main body 10 such that the outer periphery of base part 12 and the inner periphery of the opening of container main body 10 make intimate contact with each other, and thus the reduced pressure state in container main body 10 is maintained. It is to be noted that, as illustrated in FIG. 1B, base part 12 may hermetically seal disperse medium housing part 16 and sample housing part 14 of second member 11B described later. Also in this case, when the inner periphery of sample housing part 14 and the outer periphery of base part 12 make intimate contact with each other, and/or when the inner periphery of disperse medium housing part 16 and the outer periphery of base part 12 make intimate contact with each other, the inside of disperse medium housing part 16 and sample housing part 14 are hermetically sealed.

The shape of base part 12 is not limited as long as container main body 10, sample housing part 14 and disperse medium housing part 16 can be hermetically sealed as described above, and the shape of base part 12 is appropriately selected in accordance with the shape of container main body 10, sample housing part 14, and/or disperse medium housing part 16. The material of base part 12 is not limited; however, in view of ease of fitting to container main body 10 or the like, and ease of intimate contact with container main body 10, it is preferable that the portion which makes contact with container main body 10, sample housing part 14, and/or disperse medium housing part 16 be composed of an elastic member such as rubber. Base part 12 may be composed of, for example, a combination of a resin mold and an elastic member; however, it is preferable that the entirety of base part 12 be composed of an elastic member in view of manufacturing efficiency and the like.

First member 11A further includes sample injection hole 13 for injecting a sample into base part 12, and disperse medium injection hole 15 for injecting a disperse medium into base part 12 as illustrated in FIG. 3B. Sample injection hole 13 is a hole for introducing a sample from the outside of emulsion production member 11 to sample housing part 14 described later. Disperse medium injection hole 15 is a hole for introducing a disperse medium from the outside of emulsion production member 11 to disperse medium housing part 16 described later. Normally, injection of a sample to sample injection hole 13, and/or injection of a disperse medium to disperse medium injection hole 15 is performed with a hollow needle.

It is preferable that, in unused state, sample injection hole 13 and disperse medium injection hole 15 of emulsion production member 11 be not communicated with the outside of base part 12. If sample injection hole 13 and disperse medium injection hole 15 are communicated with the outside of base part 12, it is difficult to maintain the reduced pressure state of container main body 10. In view of this, it is preferable that the surfaces of sample injection hole 13 and disperse medium injection hole 15 of unused emulsion production member 11 be covered with a sheet (not illustrated) or the like which can be pierced with a needle, or that a cap (not illustrated) which closes sample injection hole 13 and disperse medium injection hole 15 be attached. On the other hand, if air enters container main body 10 from sample injection hole 13 and/or disperse medium injection hole 15 during use of emulsion production member 11, it is difficult to maintain the reduced pressure state of container main body 10. In view of this, it is preferable that the diameters of sample injection hole 13 and disperse medium injection hole 15 be equal to or smaller than the outer diameter of the hollow needle for injecting a sample and/or disperse medium.

The positions of sample injection hole 13 and disperse medium injection hole 15 in first member 11A are appropriately selected in accordance with the position of sample housing part 14 and/or the position of disperse medium housing part 16 described later. While the first member includes one sample injection hole 13 and one disperse medium injection hole 15 in the present embodiment, the first member may include a plurality of sample injection holes 13 and a plurality of disperse medium injection holes 15. In addition, the method of forming sample injection hole 13 and disperse medium injection hole 15 in base part 12 is not limited, and a publicly known method for forming a through hole may be adopted.

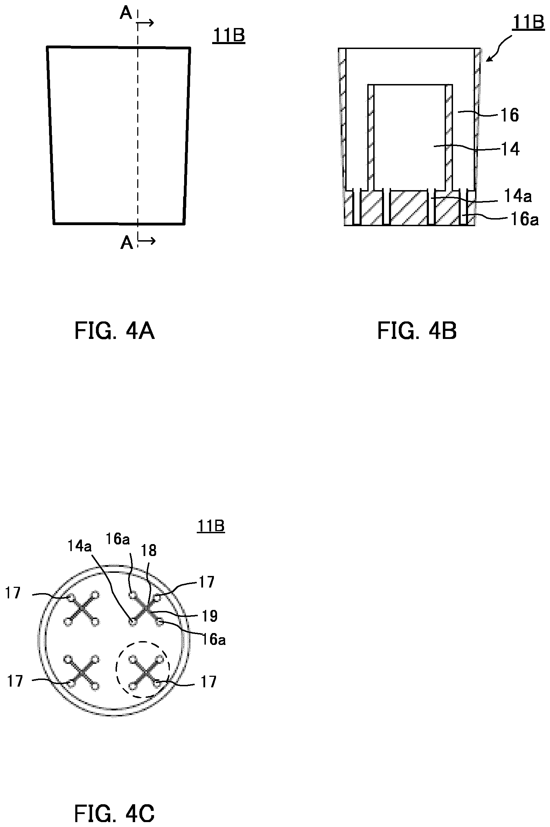

FIG. 4A is a front view of second member 11B of emulsion production member 11, FIG. 4B is a sectional view of the second member 11B taken along line A-A, and FIG. 4C is a bottom view of the second member 11B. As illustrated in FIG. 4B, second member 11B of emulsion production member 11 includes sample housing part 14 and disperse medium housing part 16. The shapes of sample housing part 14 and disperse medium housing part 16 are not limited as long as a sample and a disperse medium can be temporarily housed. In the present embodiment, sample housing part 14 is formed in a columnar shape at a center portion of second member 11B, and disperse medium housing part 16 is formed in an annular shape on the outer periphery side in second member 11B. The positions of sample housing part 14 and disperse medium housing part 16 are not limited, and disperse medium housing part 16 may be disposed at a center, for example.

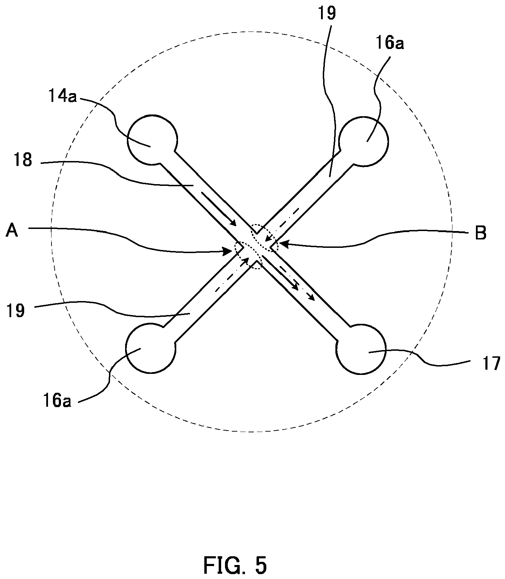

As illustrated in the bottom view of FIG. 4C, second member 11B further includes sample channel groove 18 and disperse medium channel groove 19 for carrying a sample and a disperse medium in a desired direction. FIG. 5 is a partially enlarged view of the portion indicated with the broken line in FIG. 4C. As illustrated in FIG. 4C and FIG. 5, sample channel groove 18 and disperse medium channel groove 19 are grooves formed in the bottom of the second member, and when second member 11B and film 11C described later are joined together, the openings of sample channel groove 18 and disperse medium channel groove 19 are closed. Then, the region surrounded by sample channel groove 18 and film 11C serves as a channel of the sample (hereinafter referred to also as "sample channel"), and the region surrounded by disperse medium channel groove 19 and film 11C serves as a channel of the disperse medium (hereinafter referred to also as "disperse medium channel").

Here, one end of sample channel groove 18 is connected with sample housing part 14 at channel 14a, and the other end of sample channel groove 18 is connected with film 11C described later at outlet 17. On the other hand, one end of disperse medium channel hole 19 is connected with disperse medium housing part 16 at channel 16a, and the other end of disperse medium channel hole 19 is connected with sample channel groove 18.

In FIG. 5, the solid line arrow indicates the flow direction of the sample, and the dashed line arrow indicates the flow direction of the disperse medium. As illustrated in FIG. 5, the sample flows in sample channel groove 18 from channel 14a communicated with sample housing part 14 toward outlet 17. The disperse medium flows in disperse medium channel groove 19 from channel 16a communicated with disperse medium housing part 16 toward the sample channel side opening (the portions indicated by A or B in FIG. 5) of disperse medium channel groove 19, and flows into sample channel groove 18. At this time, the flow of the sample in sample channel groove 18 is divided by the disperse medium, and the sample becomes minute droplets. Then, the dispersed liquid droplets are dispersed with the disperse medium, and discharged from outlet 17 of film 11C described later.

In the present embodiment, two disperse medium channel grooves 19 are connected with one sample channel groove 18, and the opening of one disperse medium channel groove 19 on sample channel groove 18 (the region indicated by A in FIG. 5) and the other opening of disperse medium channel groove 19 on sample channel groove 18 side (the region indicated by B in FIG. 5) face each other with sample channel groove 18 therebetween. With this configuration, in the region near the openings (the regions indicated by A and B in FIG. 5) of disperse medium channel grooves 19 on sample channel groove 18 side, shearing forces from both sides are exerted on the sample flow. Accordingly, the sample flow is easily divided by the disperse medium, and the sample becomes minute droplets having an extremely small particle size.

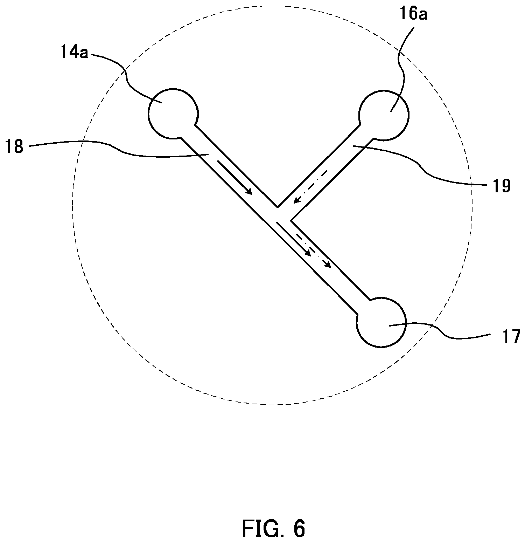

Note that sample channel groove 18 and disperse medium channel groove 19 may be connected with each other in a T-shape as illustrated in FIG. 6. In this case, a shearing force in one direction is exerted on the sample flow. Such a shearing force can also sufficiently divide the sample flow, and emulsion in which minute droplets of the sample are dispersed with the disperse medium is produced as described above.

While sample channel groove 18 and disperse medium channel groove 19 are linearly formed in the present embodiment, the grooves may be formed in a curved line shape. In addition, sample channel groove 18 and disperse medium channel groove 19 may be partially or entirely tilted to the surface of film 11C.

While second member 11B includes four pairs of sample channel groove 18 and disperse medium channel groove 19 as illustrated in FIG. 4C in the present embodiment, the numbers of sample channel groove 18 and disperse medium channel groove 19 of second member 11B is not limited to this.

Here, it is preferable that the amount (volume) of the disperse medium which flows into the sample channel be greater than the amount (volume) of the sample which flows into the sample channel. If the amount of the disperse medium is excessively small, produced minute droplets of the sample might make contact with each other so as to be unified. The amount of the sample which flows into the sample channel is adjusted by the diameter of channel 14a formed in sample housing part 14, the height of sample channel groove 18, the width of sample channel groove 18 and the like. Also, the amount of the disperse medium which flows into the sample channel is adjusted by the diameter of channel 16a formed in disperse medium housing part 16, the height of disperse medium channel groove 19, the width of disperse medium channel groove 19 and the like.

In addition, film 11C described later can be joined to second member 11B by thermal welding. In view of this, preferably, second member 11 has a bottom having a flat external shape slightly larger than the external shape of film 11C. With second member 11B having such a bottom, a uniform pressure can be exerted on the entirety of the bottom of second member 11B which serves as the joint surface when joining film 11C to second member 11B, and thus stable joining can be achieved. In addition, it is possible to prevent protrusion of film 11C from the bottom of second member 11B which impede insertion of emulsion production member 11 as a stopper into container main body 10 of sample collection tube 100.

Here, as long as sample housing part 14, disperse medium housing part 16, sample channel groove 18 and disperse medium channel groove 19 can be formed in desired shapes, second member 11B is not limited and may be a publicly known resin mold.

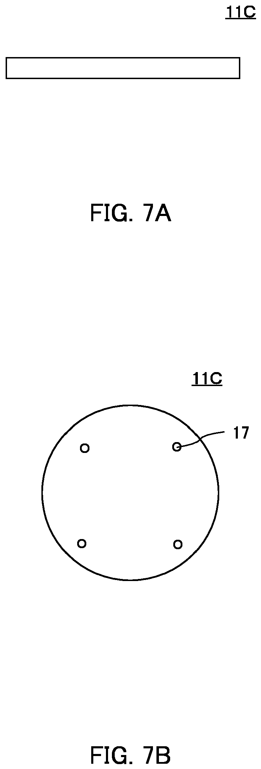

FIG. 7A is a front view illustrating film 11C of emulsion production member 11 of the present embodiment, and FIG. 7B is a plan view illustrating film 11C. Film 11C is a member configured to be attached on the bottom surface of second member 11B so as to close the openings of sample channel groove 18 and disperse medium channel groove 19 of second member 11B, and to discharge, toward container main body 10 side, the emulsion in which the sample in the form of minute droplets is dispersed with the disperse medium.

As illustrated in FIG. 7B, film 11C may be a plate-shaped member having outlet 17 (through hole). As necessary, film 11C may include grooves (not illustrated) at positions corresponding to sample channel groove 18 and disperse medium channel groove 19 of second member 11B. A protrusion (not illustrated) may be provided in the regions corresponding to sample channel groove 18 and disperse medium channel groove 19. In a case that film 11C includes protrusions corresponding to sample channel groove 18 and disperse medium channel groove 19, sample channel groove 18 and disperse medium channel groove 19 are fitted to the protrusions. In this case, since the region surrounded by sample channel groove 18 or disperse medium channel groove 19 and the top surfaces of the protrusions of film 11C serves as a sample channel or a disperse medium channel, and the sample channel and/or the disperse medium channel is sufficiently hermetically sealed with the protrusions, the sample and/or the disperse medium hardly leaks. Further, in a case that film 11C includes the above-mentioned protrusion, ease of alignment of film 11C and second member 11B is advantageously increased.

The material of film 11C is not limited as long as film 11C can sufficiently make intimate contact with second member 11B. For example, it is possible to adopt a publicly known resin film, or a film made of an elastic member such as rubber.

### Usage of emulsion production member

Usage of emulsion production member 11 of the present embodiment is described below. While an example case that the sample is blood and the disperse medium is oil which is not compatible with blood is described below, the sample and/or the disperse medium is not limited to this.

First, as illustrated in FIG. 1, emulsion production member 11 of the present embodiment is fitted into container main body 10 to obtain sample collection tube 100. Emulsion production member 11 may be fitted into container main body 10 at atmosphere pressures, but it is preferable to fit emulsion production member 11 into container main body 10 under a reduced pressure. In this manner, a reduced pressure state can be established inside container main body 10 after emulsion production member 11 is fitted. Container main body 10 is not limited as long as container main body 10 includes an opening at one end and the opening has a shape which can make intimate contact with base part 12 of emulsion production member 11. The shape and the material of a publicly known blood collection tube or a publicly known sample tube may be adopted.

Next, disperse medium injection hole 15 of emulsion production member 11 and an oil-filled container filled with oil (disperse medium) are coupled together with a coupling tube or the like. For example, disperse medium injection hole 15 and the oil-filled container may be coupled by inserting a needle disposed at one end of the coupling tube into disperse medium injection hole 15, and inserting a needle disposed at the other end of the coupling tube into the oil-filled container. When the oil-filled container and disperse medium injection hole 15 are coupled together, the oil is moved into emulsion production member 11 by the negative pressure in container main body 10. Note that, before starting feeding of the oil, it is preferable to pinch the coupling tube with a pinchcock or the like.

On the other hand, sample injection hole 13 of emulsion production member 11 and a subject (e.g. a person subjected to blood collection) are coupled together with a coupling tube. For example, a needle disposed at one end of the coupling tube is inserted into sample injection hole 13 and a blood collection needle is inserted into a person subjected to blood collection, and thus, blood is moved to emulsion production member 11 side by the negative pressure in container main body 10. Note that, before starting feeding of blood to emulsion production member 11 side, it is preferable to pinch the coupling tube with a pinchcock or the like.

When starting production of emulsion (collection of blood), first, the pinchcock of the tube coupling the oil-filled container and disperse medium injection hole 15 is opened to feed oil to emulsion production member 11 side. Thereafter, the pinchcock of the tube coupling the person subjected to blood collection and sample injection hole 13 is opened to feed blood to emulsion production member 11 side. In this manner, oil and blood are housed into disperse medium housing part 16 and sample housing part 14 of emulsion production member 11. Then, when the oil and the blood pass through the sample channel and the disperse medium channel formed below emulsion production member 11, the blood is transformed into minute droplets by the oil, and emulsion in which the minute droplets of the blood are dispersed with oil is discharged into container main body 10.

While the oil and/or the blood is fed to emulsion production member 11 by the negative pressure in container main body 10 in the above-mentioned description, the oil and/or the blood may be fed to emulsion production member 11 side by applying pressure from the oil-filled container side, and/or the subject side.

### Effect

With the emulsion production member of the embodiment, a sample can be divided into minute droplets without modifying the sample. In particular, in a case that blood is used as the sample, the blood can be dispersed in a unit of a blood cell or a unit of a cell with oil. In a conventional blood collection tube, it is difficult to separately store blood for each blood cell or cell. Therefore, when hemolysis occurs, identification of the blood cell or the cell where the component originates becomes difficult, and thus it is difficult to analyze a component originating from a specific blood cell or a specific cell. In contrast, with the emulsion production member of the embodiment, even when hemolysis occurs and/or the component is partially altered, the components of the blood cell or the cell are not mixed with other blood cells or cell components. Accordingly, a component originating from a specific blood cell or a specific cell can be analyzed.

This application is entitled to and claims the benefit of Japanese Patent Application No. 2016-119764 filed on June 16, 2016, the disclosure each of which including the specification, drawings and abstract is incorporated herein by reference in its entirety.

### Industrial Applicability

With the emulsion production member of the embodiment of the present invention, a sample can be divided into minute droplets, and can be stored in a dispersed state with a disperse medium. Accordingly, for example, when blood is used as the sample, the sample can be stored in a unit of blood cell or cell, and therefore mixing with components originating from other blood cells or other cells is prevented even when hemolysis occurs. That is, it is possible to inspect only a component originating from a specific blood cell or a specific cell, which is very useful in immunity inspection and other researches.

### Reference Signs List

- 10: Container main body
- 11: Emulsion production member (stopper)
- 11A: First member
- 11B: Second member
- 11C: Film
- 12: Base part
- 13: Sample injection hole
- 14: Sample housing part
- 15: Disperse medium injection hole
- 16: Disperse medium housing part
- 17: Outlet
- 18: Sample channel groove
- 19: Disperse medium channel groove

## Claims

1. An emulsion production member configured to be attached to a container main body whose internal pressure is reduced, and configured to divide a sample into minute droplets and disperse the minute droplets with a disperse medium, the emulsion production member comprising:
a stopper configured to maintain the reduced internal pressure of the container main body;
a sample injection hole configured to inject the sample into the stopper;
a sample housing part disposed in the stopper and configured to house the sample injected through the sample injection hole;
a disperse medium injection hole configured to inject the disperse medium into the stopper;
a disperse medium housing part disposed in the stopper and configured to house the disperse medium injected through the disperse medium injection hole;
an outlet configured to discharge, to the container main body side, the sample housed in the sample housing part and the disperse medium housed in the disperse medium housing part;
a sample channel configured to connect between the sample housing part and the outlet and carry the sample; and
a disperse medium channel configured to connect between the disperse medium housing part and the sample channel, and configured to carry the disperse medium to divide the sample flowing in the sample channel into minute droplets by the disperse medium.

2. The emulsion production member according to claim 1,
wherein the disperse medium channel and the sample channel are provided such that two disperse medium channels are provided for one sample channel, and that an opening of one of the disperse medium channels on the sample channel side and an opening of the other disperse medium channel on the sample channel side face each other with the sample channel between the openings.

3. A sample collection tube comprising:
a container main body including an opening; and
the emulsion production member according to claim 1 or 2 attached to the opening of the container main body, wherein
a pressure inside the container main body is in a reduced state.

4. A blood collection tube comprising:
a container main body including an opening; and
the emulsion production member according to claim 1 or 2 attached to the opening of the container main body, wherein
the sample is blood.
